# EUROPEAN PATENT APPLICATION

(11) **EP 1 571 218 A1**
(43) Date of publication of application: **07.09.2005**
(21) Application number: 05004437.9
(22) Date of filing: 01.03.2005
(51) Int. Cl.: C12N 15/82, A01H 5/00, A61K 39/00

(54) **Animal immunocontraceptives expressed in plants and uses thereof**

(30) Priority: 01.03.2004 US 790291
(71) Applicant: RESEARCH DEVELOPMENT FOUNDATION, Nevada 89703 (US)
(72) Inventor: Kitto, Barrie G., Austin, TX 78731 (US); Hirschhorn, Daniel C., New York, NY 10021 (US)
(74) Representative: Bösl, Raphael Konrad

(57) **Abstract**

The present invention provides immunocontraceptive vaccines comprising a genetically engineered plant that has been modified to produce the sperm-specific protein lactate dehydrogenase-C (LDH-C). When animals such as rodents eat this plant, their immune systems produce antibodies that attack their sperms. Not only would the males have less viable sperm, the females would also have antibodies to sperm entering their reproductive systems. Immunocontraception is an attractive method for reducing the population size of animals with high fecundity, and sterilizing animals using such immunocontraceptives can reduce targeted animal populations to acceptable levels in an efficient, cost-effective, humane and, importantly, a species-specific manner.

## Description

### BACKGROUND OF THE INVENTION

### Field of the Invention

The present invention relates generally to the field of immunocontraceptives. More specifically, the present invention relates to methods of controlling animal populations using immunocontraceptives expressed in plants.

### Description of the Related Art

Rodent pests have infiltrated human activities and caused great economic and social impact. As pests rodents are equally as destructive as insects. They can cause massive destruction of crops and foodstuffs and spread fatal diseases to people and domestic animals. The so-called comensal rodents include the Norway rat (*Rattus norvegicus*), the roof or house rat (*Rattus rattus*), and the several sub-species of mice *Mus musculus* (*domesticus, spretus, mecedonicus, hortulanus, molossinus,* and *castaneous*).

According to estimates by the Food and Agricultural Organization of the United Nations, rodents destroy 40 million tons of food annually. It has been estimated that some 1/3 to 1/5 of the world's crops are being directly or indirectly destroyed by commensal rodents.

In industrialized countries with an overproduction of crops, the damage is inflicted primarily in storage facilities where losses are primarily economic. In contrast, in developing nations where starvation is prominent, crops are being destroyed in the field as well as in storage and rodent control can sometimes make the difference between life and death. A storage facility can be severely decimated by a rodent infestation. An adult rat can consume 20-30g of grain per day; this means that just 100 rats on a farm will consume at least a ton of food intended for humans or animal fed.

The damage to crop is usually overshadowed by the very serious contamination of grain with feces, urine, and hair. A rat produces on average 40 droppings a day. At this rate, ten rats will produce 146,000 droppings a year. The same amount of rats can produce 54 liters of urine. If only a few droppings or a small amount of urine finds its way to food it would most likely be rejected. In addition, rodent carcasses killed by poisons can render useless entire reservoirs of grain and other crops.

Rats, mice, and other rodents are capable of carrying and transmitting a variety of diseases to human and other animals. The viruses rodents transmit can cause diseases in man with fatality rates of 50% or more. Some examples include Venezuelan equine encephalitis, tick-born encephalitis, rodent-borne hemorrhagic fevers (Argentine, Bolivian, and Venezuelan), Lassa virus, and Hemorrhagic fever with renal syndrome.

Rodents can transmit a variety of rickettsial diseases using vectors such as ticks, mites, fleas, or mosquitoes. The principal rickettsial diseases that inflict human populations include louse-borne typhus (*Rickettsia prowazeki*), Rocky Mountain spotted fever, Boutonneuse fever, tustsugamushi diseases (Scrub typhus), and murine typhus (*Rickettsia typhi*). Rodents are also reservoirs of a great number of bacterial diseases. In fact, they are particularly known for carrying bacteria such as *Salmonella,* plague, Lyme disease, *Borrelia,* and leptospirosis.

Mechanical/physical rodent control includes shooting, flooding burrows with water, use of ultrasound or electromagnetic waves. Shooting and water flooding are certainly ineffective. Using high tech devices such as ultrasound or electromagnetic waves is not only an extremely expensive alternative but also has never been shown to produce consistent results. Trapping will produce results under very limited conditions such as a house. However, trapping does not protect crops and it is only used in conjunction with chemical rodenticides to remove surviving animals from treated areas.

Many unsuccessful attempts to introduce pathogens for pest control have been attempted. The myxoma virus was introduced into Australia to control rabbits some 50 years ago. Similar approaches using *Salmonella enteritis* to control rodents in many countries have been attempted since the turn of the century in the form of baits called *Ratin*.

A genetical method of rodent control refers to altering the normal gene pool within a population by introducing a deleterious gene or introducing sterile males. For example, introducing genes such as the one causative of the Gruneber Lethal Syndrome that results in the death of 25% of the offspring before puberty has been explored. However, natural selection will operate to eliminate such genes from the population.

There are three types of chemicals used for controlling rodents: repellents or attractants, rodenticides, and chemosterilants. Repellents, although helpful in some situations, are not species-specific and can potentially render food or goods with repugnant odor or taste. Attractants have long been used in combination with either traps, poisons, and/or, chemosterilants.

Chemical poisons or rodenticides are the most widely used and efficient of all available methods for rodent control. The introduction of these agents provided society with an extremely powerful tool in the battle against rodents. The rodents, however, started to become resistant to the poisons. There is obviously a selection pressure favoring genetic resistance to a poison that can be rapidly fixed in the population. Eventually, certain poisons were no longer effective to control infestation at all and new chemicals have to be developed. Thus, the use of chemical poisons is not an effective strategy in the long-term because rodents breed rapidly and those that were killed are readily replaced.

It has been suggested that reducing fertility is a more preferable and effective method to control rodents and other mammalian pests. In rodent and other vertebrate pest control, sterilization is considered to have more potential and more impact on a population than conventional killing approaches.

The purpose of an anti-fertility vaccine or immunocontraceptive is to induce immune response against proteins from the reproductive system, thereby leading to infertility. Perhaps the widest application of contraceptive vaccines to date is for the control of rodents and other mammalian species. An effective immunocontraceptive could alleviate some of the burdens of the technologies used today. A successful contraceptive vaccine will have to block fertilization or embryonic development, be species specific, and provoke a sustained immune response. Additionally, an effective mechanism which is cost effective to manufacture and administer for transmitting the vaccine safely throughout the target population must be found.

A fundamental issue in the development of immunocontraceptive agents for the control of wild species is the method of delivery. The prior art is deficient in efficient methods of delivering immunocontraceptives useful in controlling animal populations such as rodents. The present invention fulfills this long-standing need and desire in the art by providing delivery system involving transgenic plants.

### SUMMARY OF THE INVENTION

The present invention discloses immunocontraceptives for mammalian pest control. The contraceptive agent is based on the model sperm antigen lactate dehydrogenase C (LDH-C). The basic premise is to provide adequate delivery systems for the immunocontraceptive agent such that a sterilizing immune response will be elicited. The contraceptive agents disclosed herein have the advantages of being more effective and humane than the current methods, as well as environmentally friendly, cost effective, and species-specific.

Recombinant lactate dehydrogenase C was expressed in transgenic plants such as *Arabidopsis* and tobacco. Purified lactate dehydrogenase C from tobacco leafs was capable of eliciting sterilizing antibody production in animals. Because the antibodies react to sperm, the instant contraceptive agent affects the fertility of both males and females. Not only would the males have less viable sperms, the females would also have antibodies to the sperms entering their reproductive systems. In this case, the plant-vaccines would be delivered orally. The contraceptive agents could be prepared into a bait formulation that could be strategically placed among the targeted animal populations. The animals would be sterilized upon consumption of the vaccine, thus promoting population control.

In one embodiment of the present invention, there is provided a genetically modified plant that expresses an immunocontraceptive comprising an egg- or sperm-specific polypeptide. The contraceptive agent is useful in controlling the size of an animal population by inducing sterility in animals that have ingested the genetically-engineered plant materials.

In another embodiment of the present invention, there are provided methods of using these contraceptive agents to control the population size of animals that has reached pest levels.

Other and further aspects, features, and advantages of the present invention will be apparent from the following description of the presently preferred embodiments of the invention. These embodiments are given for the purpose of disclosure.

### BRIEF DESCRIPTION OF THE DRAWINGS

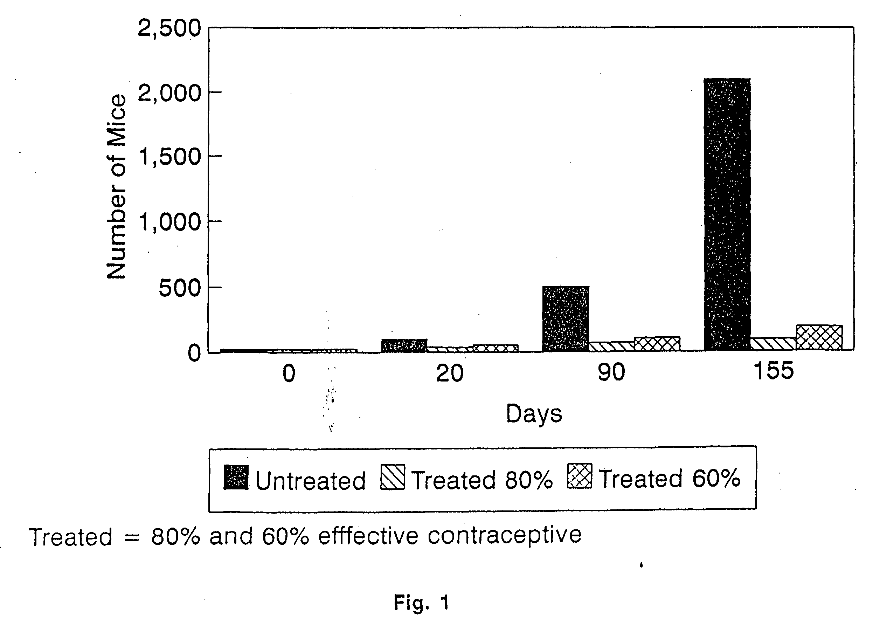

**Figure 1** shows a modeling of rodent population size after treatment with 60% or 80% effective contraceptive. The constraints used were: initial population = 20 mice (10 female, 10 male); average lifespan = 1 year; gestation time = 20 days; fecundity, 5 litters/year, 8 offspring/litter; time to sexual maturity from birth = 45 days.

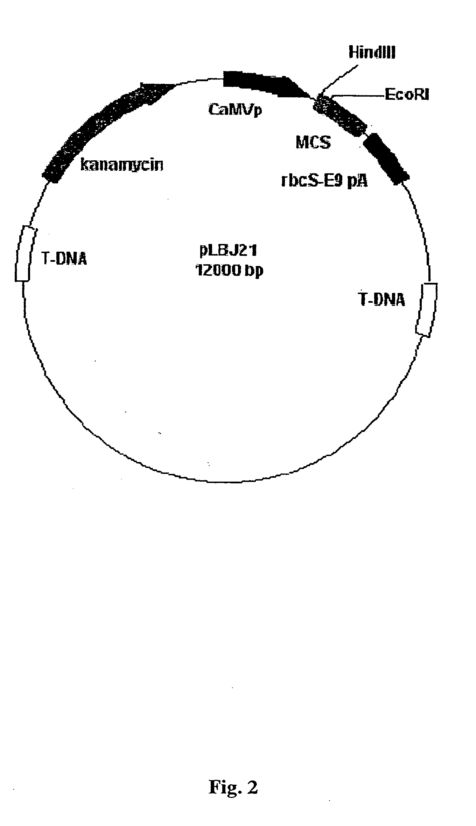

**Figure 2** is a map of pLBJ21.

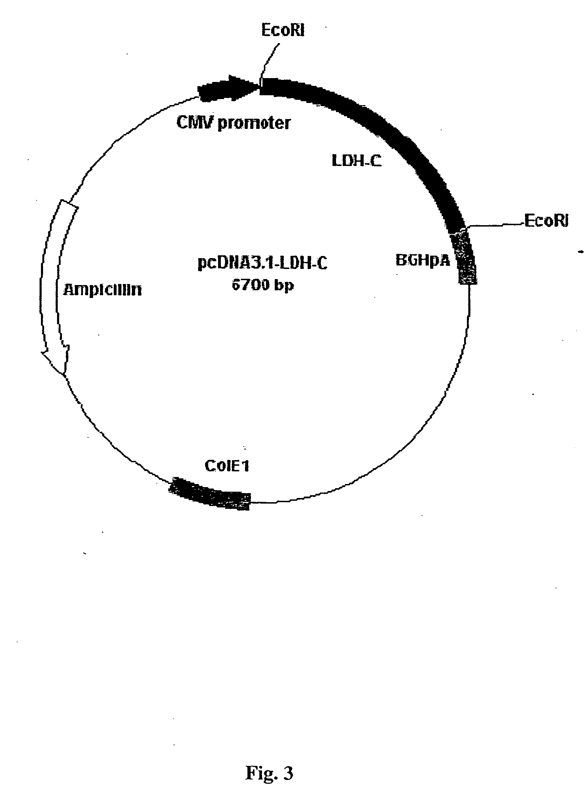

**Figure 3** is a map of pcDNA3.1-LDH-C.

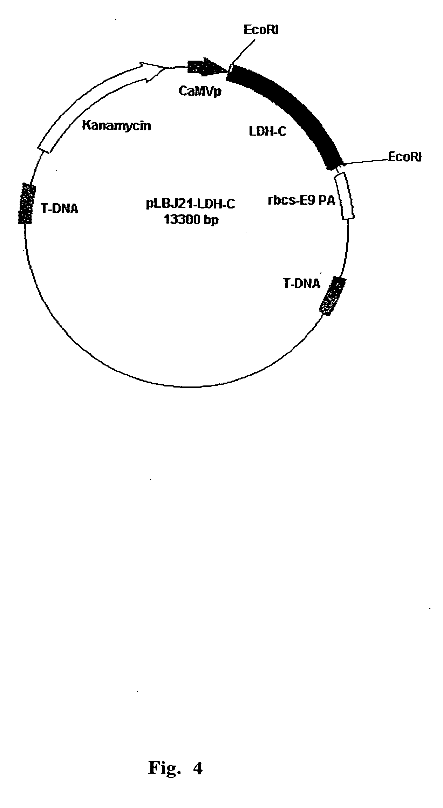

**Figure 4** is a map of pLBJ21-LDH-C. The plasmid is a modified *Agrobacterium* plasmid capable of expressing lactate dehydrogenase-C (LDH-C) in plants.

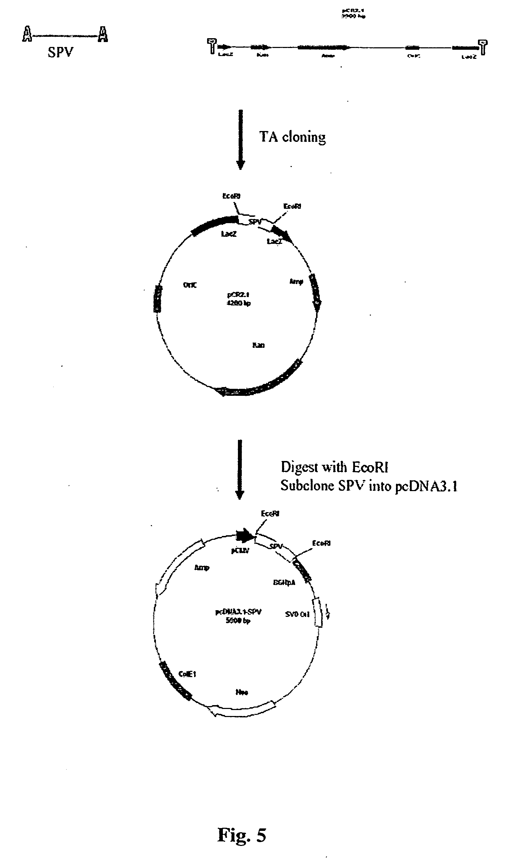

**Figure 5** shows the cloning strategy of SPV (Short Peptide Vaccine).

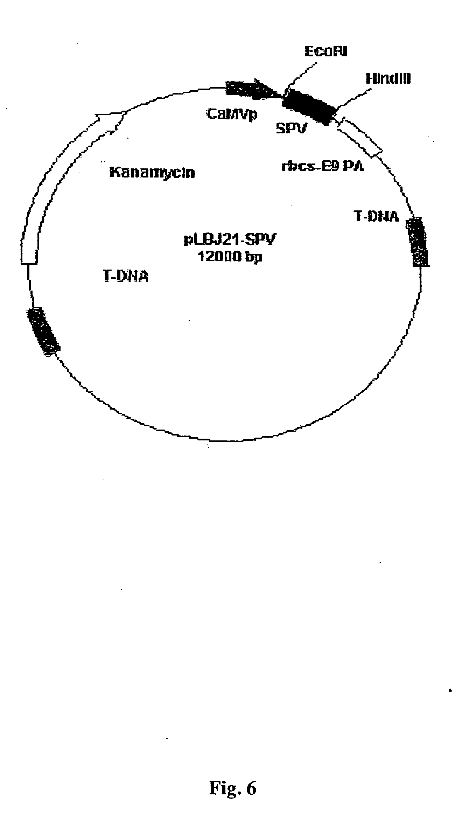

**Figure 6** is a map of pLBJ21-SPV.

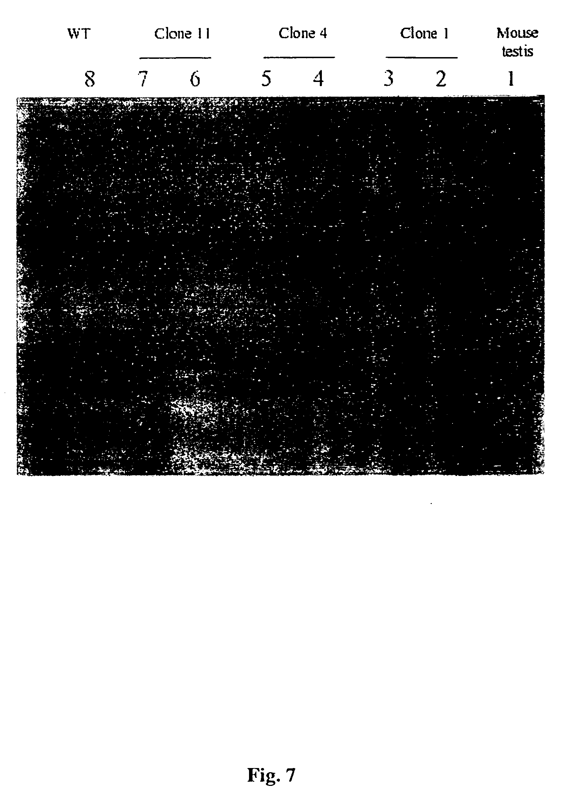

**Figure 7** shows expression of lactate dehydrogenase-C in *Arabidopsis thaliana*: Extracts from plants of clones 1, 4, and 11 or wild type (wt) were prepared for western blots. The immunoblots were probed with a rabbit anti mouse lactate dehydrogenase-C. Mouse testes were used as the postitive control.

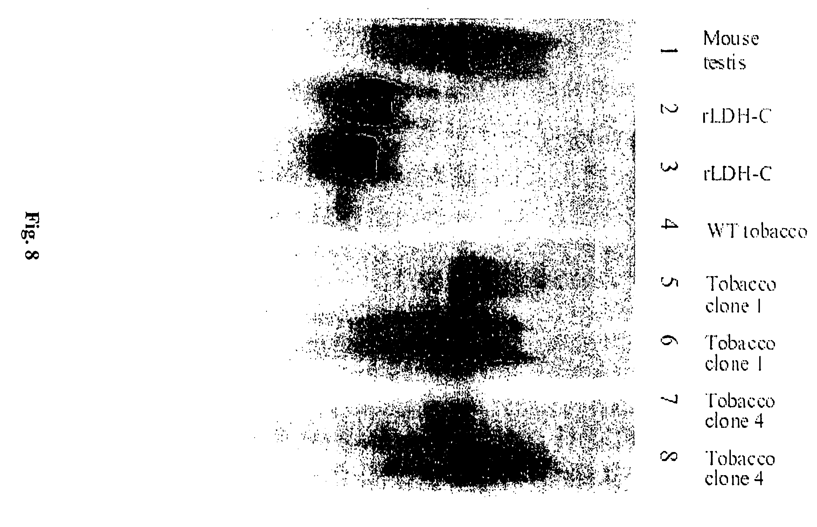

**Figure 8** shows native gel of extracts stained with a tetrazolium salt. Lane 1, mouse testis extract; lane 2, rLDH-C 10µl; lane 3, rLDH-C 5µl; lane 4, 30µl wild type tobacco extract; lane 5, 2µl extract from transgenic clone 1; lane 6, 30µl extract from transgenic clone 1; lane 7, 2µl extract from transgenic clone 4; lane 8, 30µl extract from transgenic clone 4. The transgenic plants expressed active lactate dehydrogenase-C by comparison to mouse testis extracts or to the wild type enzyme. The small band on the wild type lane was believed to be contamination from an overloaded rLDH-C adjacent lane. Recombinant lactate dehydrogenase-C migration was further than native lactate dehydrogenase-C since the recombinant protein was genetically fused to a highly positively charged histidine tag.

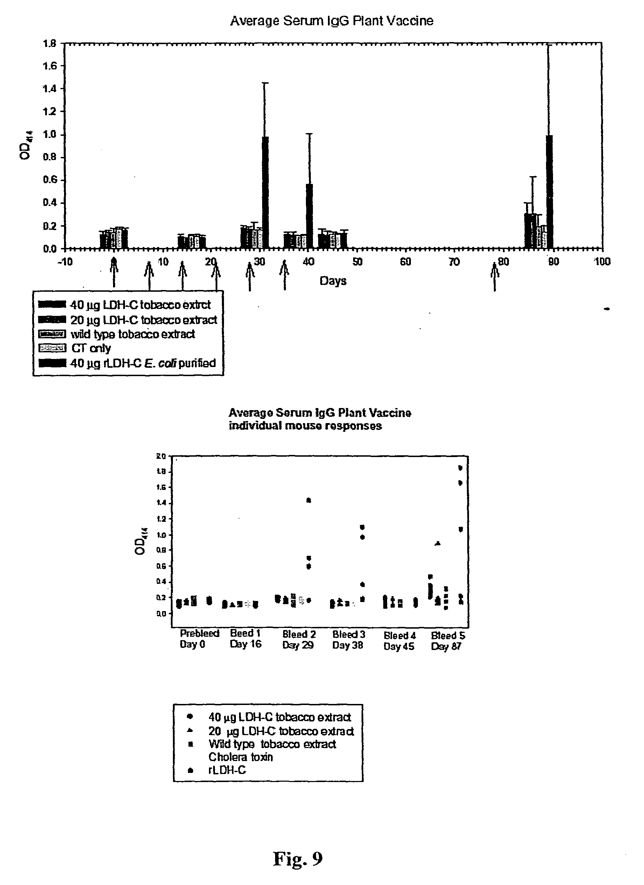

**Figure 9** shows serum IgG responses to oral immunization of tobacco extracts. The arrows point to the days where the animals were vaccinated. The bars are the average response of about 10 or 5 animals, whereas the lower graph shows the individual responses. Serum was collected on the days shown. After the last immunization a specific immune response can be seen in both 40 and 20 µg lactate dehydrogenase-C dose. A modest cross reaction response was seen in wild type tobacco extracts due to the large amount of antigenic protein that was contained in the extracts.

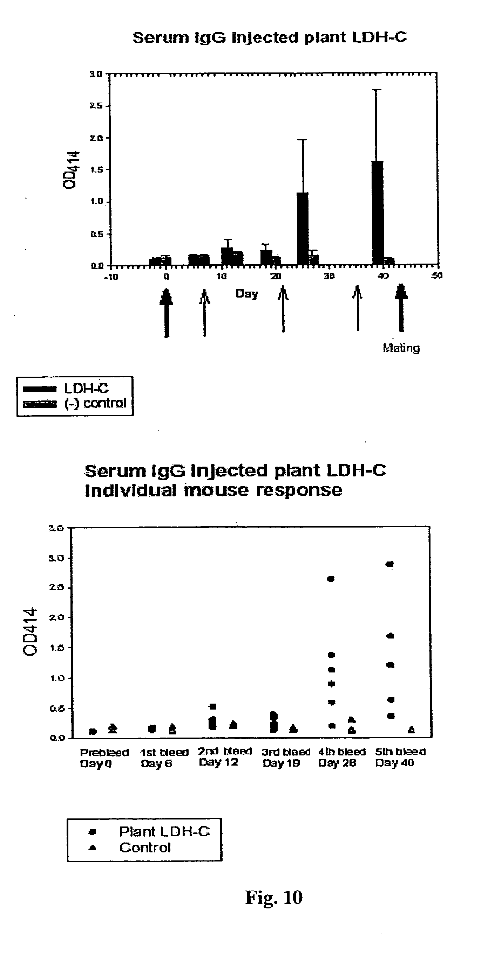

**Figure 10** shows serum IgG responses to lactate dehydrogenase-C (LDH-C) purified from to lactate dehydrogenase-C bacco leaves. The dark arrow represents 40 µg of plant LDH-C injected intraperitonealy. The lighter arrows are 20 µg boost. The red line is the date the animals were mated. The upper graph represents the average of a group of mice injected with pure lactate dehydrogenase-C emulsified in Titermax Gold or just the adjuvant as negative control. The lower graph represents the response of individual mouse from each group.

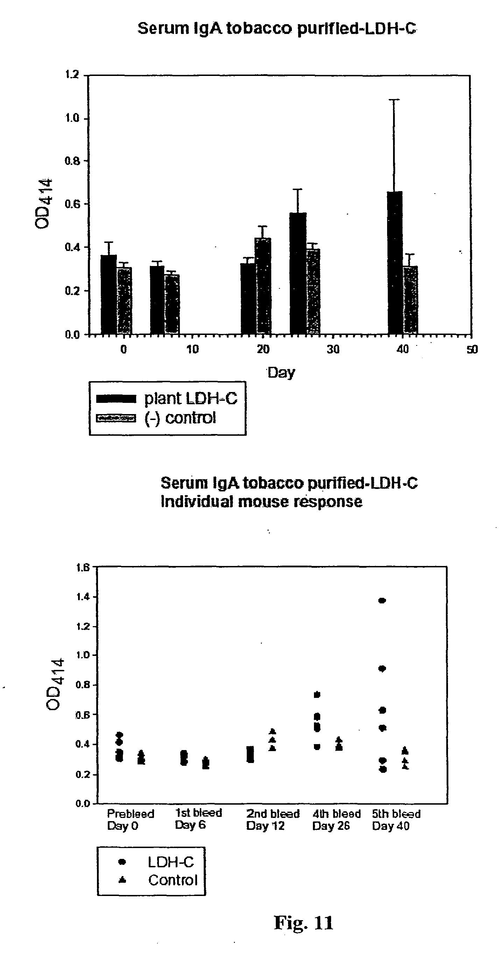

**Figure 11** shows serum IgA responses to lactate dehydrogenase-C (LDH-C) purified from tobacco leaves. The upper graph represents the average of a group of mice injected with purified lactate dehydrogenase-C emulsified in Titermax Gold or just the adjuvant as negative control. The lower graph represents the response of individual mouse from each group.

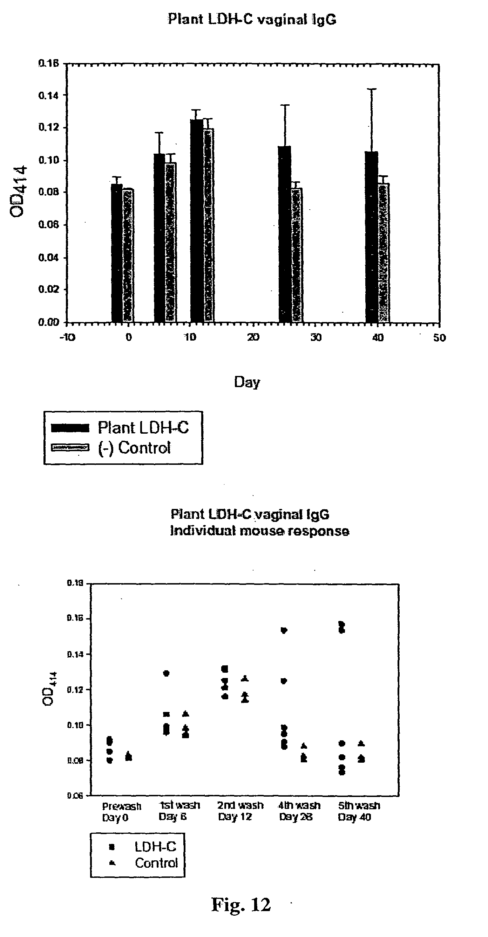

**Figure 12** shows vaginal IgG responses to lactate dehydrogenase-C (LDH-C) purified from tobacco leaves. The upper graph represents the average of a group of mice injected with purified LDH-C emulsified in Titermax Gold or just the adjuvant as negative control. The lower graph represents the response of individual mouse from each group.

### DETAILED DESCRIPTION OF THE INVENTION

As used herein, "immunocontraceptive" refers to an immunogenic composition comprising an antigen that can induce immune responses against the cells of an animal's reproductive system, thereby leading to loss of fertility in the treated animal.

The present invention discloses development of new methods for the control of animal populations that reach pest proportion. The present invention incorporates recently developed immunocontraception technologies. The basic premise of immunocontraceptives is to orally immunize animals by using bait formulations containing proteins from the animals' own reproductive system so that immune responses that block fertilization are induced in the animals after ingestion of such baits. Immunocontraception is an attractive method for reducing the population size of animals with high fecundity, and it is believed that sterilizing animals using such immunocontraceptives can reduce targeted animal populations to acceptable levels in an efficient, cost-effective, humane and, importantly, a species-specific manner.

Using some simplifying assumptions in a modeling of contraceptive effects on mouse populations, some calculations were made of how vaccines of 60% and 80% effectiveness might affect the population growth of a small starting population of mice in a habitat without resource constraints. This is a circumstance which might exist when mice initially infest a granary. The results of such modeling are depicted schematically in Figure 1, dramatically illustrating how an oral contraceptive of even 60% effectiveness can alter the rate of mouse population growth. The constraints used were: initial population = 20 mice (10 female, 10 male); average lifespan = 1 year; gestation time = 20 days; fecundity, 5 litters/year, 8 offspring/litter; time to sexual maturity from birth = 45 days.

Although the use of a contraceptive vaccine is a new approach to rodent control, the concept of bait borne vaccines is an established practice. For example, the Texas Department of Health routinely airdrops food pellets containing rabies vaccine to control this disease in gray foxes and coyotes.

### Lactate Dehydrogenase C4 As An Antigen For Immunocontraception

All vertebrate tissue contains lactate dehydrogenases (EC 1.1.1.27). This tetrameric protein has multiple isozymes assembled primarily by two subunits, A and B, that can form 5 different molecular forms. Lactate dehydrogenase-C4 (LDH-C4) is a sixth isozyme of lactate dehydrogenase that exclusively expresses in the testis of mammals and other animal species. It represents the most predominant form in the testis and is readily detectable in mature testes displaying active spermatogenesis. The enzyme does not express in other tissue in males and it is completely absent in females. Immunofluorescent studies reveal a remarkable spatio-temporal regulation of the *ldh-c* gene where it is present in spermatocytes, spermatids, and spermatozoa but not in other non-germinal elements. LDH-C4 differs from the other lactate dehydrogenase isozymes in amino acid composition and in its catalytic properties. Most interestingly, sera raised against lactate dehydrogenase-C do not cross-react with the A or B subunits. Early experiments demonstrated that rabbit antiserum to mouse lactate dehydrogenase-C4 was shown to cross react with LDH-C4 from other species but never with any isozyme composed of LDH-A or B subunits.

The first reports that lactate dehydrogenase-C can be used as a contraceptive agent come from studies where antibodies were passively transferred to animals. In this experiment, pregnancy suppression occurred in mice treated with rabbit anti-lactate dehydrogenase-C serum (Goldberg, 1972). Subsequent studies indicated that active immunization of purified lactate dehydrogenase-C produced infertility in female mice, rabbits, and baboons (Lerum and Goldberg, 1974; Goldberg, 1973; Goldberg et al., 1981). Immunized female mice produced 60 pups as opposed to 96 for the control mice after 50 matings (Lerum and Goldberg, 1974). In experiments with rabbits the fertility of the animals was reduced by 60% when mated 40 times (Goldberg, 1973). Lastly, immunized female baboons produced 27 offspring as opposed to 80 by control animals after 30 matings (Goldberg et al., 1981). The contraceptive effects in these model animals correlated with the presence of a specific immune response. Once the immune response faded away, the fertility of the animals reverted to normal fertility levels, proving that the contraceptive effect was reversible. Moreover, this form of contraception showed no impairment of embryonic development or damage to the female reproductive tract.

The availability of complete amino acid sequence information and high-resolution crystal structure for the lactate dehydrogenase-C4 antigen (Hogrefe et al., 1987) has allowed for B-cell epitope mapping of the molecule and subsequent synthesis of species-specific immunodominant epitope peptides. Immunodominant epitopes of lactate dehydrogenase-C4 can be identified by computer algorithms for B-cell epitope prediction (Van Regenmortel and Daney de Marcillac, 1998). The predicted epitopes can be corroborated by sequence comparison with related mammalian lactate dehydrogenase-C immunodominant epitopes. The human, baboon, mouse, and rabbit share the most immunodominant epitope 5-15 amino acids at the N-terminus of the molecule (O'Hern et al., 1995; O'Hern et al., 1997). Other examples of lactate dehydrogenase-C4 antigenic domains include amino acids 5-17, 44-58, 61-77, 97-110, 101-115, 180-210, 211-220, 231-243, 283-306, and 307-316.

A peptide vaccine consisted of a lactate dehydrogenase-C4 epitope chemically linked to a diphtheria toxoid was tested in rabbits (Wheat et al., 1985). The diphtheria toxoid was used with the double purpose of carrier protein and adjuvant. The peptide was further adapted for baboon uses by using the human sequence, and up to 75% reversible contraception was shown in 15 female baboons (O'Hern et al., 1995). A completely synthetic lactate dehydrogenase-C4 peptide vaccine consisting of just 39 amino acids coupled to a "promiscuous" T-cell epitope from tetanus toxin was capable of reducing fertility by 62% in female baboons (O'Hern et al., 1997). A promiscuous T-cell epitope is a short sequence capable of providing a T-helper response needed for antibody production in a wide range of species and multiple genetic backgrounds.

Therefore, full length as well as antigenic fragments of the lactate dehydrogenase-C4 (LDH-C4) antigen can be incorporated into the immunocontraceptive of the present invention. Table 1 shows a comparison of amino acids 1-15 of lactate dehydrogenase-C4 from several species. From this table, it can be inferred that this particular sequence is divergent enough to provide some level of species specificity.

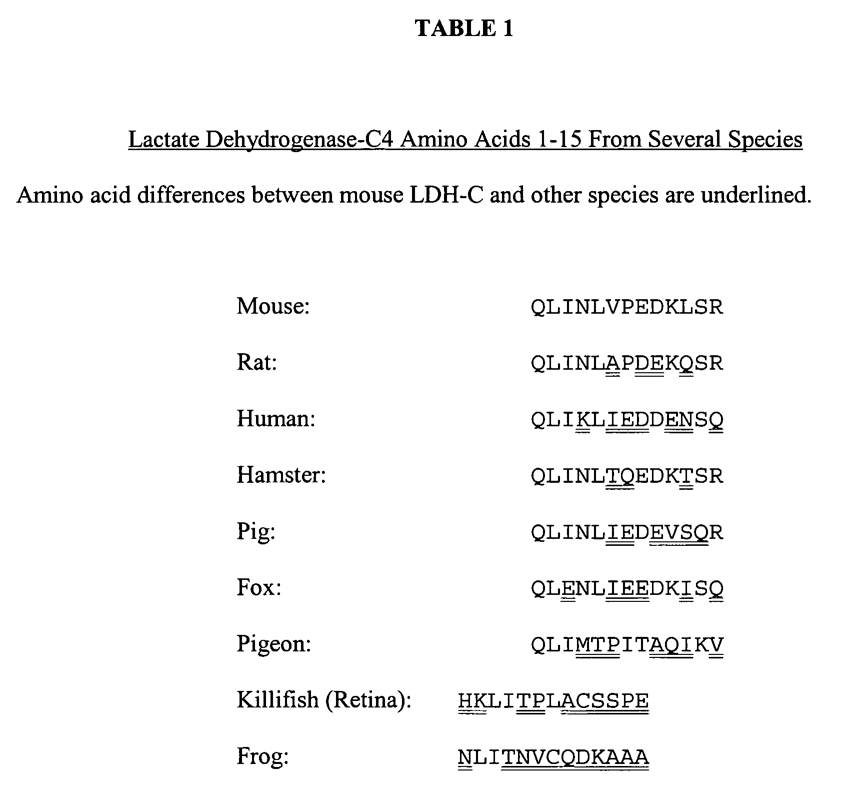

As used herein, "fragment," as applied to a polypeptide, will ordinarily be at least 8 residues, more typically at least 40 residues in length, but less than the entire, intact sequence. Fragments of the lactate dehydrogenase-C4 protein can be generated by methods known to those skilled in the art, e.g., by enzymatic digestion of naturally occurring or recombinant lactate dehydrogenase-C4 protein, by recombinant DNA techniques using an expression vector that encodes a defined fragment of lactate dehydrogenase-C4, or by chemical synthesis.

Fragment of lactate dehydrogenase-C4 have been synthesized in combination with additional elements such as diptheria toxoid or a promiscuous T-cell epitope of tetanus toxin and shown to have comparable contraceptive effectiveness to lactate dehydrogenase-C4 in several species (O'Hern et al., 1995, 1997). Other carriers considered include the use of complete cholera toxin, its beta subunit, or a genetically or chemically detoxified form of the toxin. This agent has been shown to be the strongest mucosal adjuvant and can be more adequately used when oral and mucosal vaccines are considered (Fujihashi, 2002). In a similar fashion, mucosal adjuvants from bacterial toxins or other sources could be used (Piazza, 2001).

One of ordinary skill in the art would recognize that the present invention is equally applicable to other animal or pest populations. Human activities, while driving some species to extinction or the brink of extinction, have provided a safe haven for many other species to proliferate to levels where they can pose great danger. Some of these species have become a menace to local flora, fauna, or, paradoxically, to humans themselves. There are multiple examples in many parts of the world where mammals have reached pest densities. For example, the white-tailed dear (*Odocoileus virginianus*) has been a menace in North America for almost a century. Many authors have proposed and tested the use of contraceptive vaccines for controlling vertebrate pests including deer (Kirkpatrick et al., 1997). In fact, many vertebrate pests share many characteristics with the rodents and most models developed for their immunocontraceptive control can be equally applied. Thus, other animals to which the present invention is applicable include any mammalian species capable of eliciting an immune response against proteins of their own reproductive system. In particular, deer, elephants, water buffalo, feral horses, foxes, urban or wild dogs, urban or wild cats, rabbits, and other potentially overpopulated species causing economic damage to society could be targeted.

Crucial to the development of a successful contraceptive bait is the selection of a proper carrier for the immunological agent. The carrier should be stable, capable of being inexpensively and efficiently produced, environmentally safe and attractive as a food to the targeted animals. Based on these requirements, the present invention uses plant materials as a vector for the immunocontraceptives.

### Plant-Based Immunocontraceptive Vaccine

Biopharmaceuticals have traditionally been produced in a variety of ways including transgenic expression systems such as cultured mammalian, bacterial, and fungal cells. Not until the past decade have plants been seriously considered as systems to manufacture bioproducts.

The use of plants as vaccine delivery systems was first demonstrated in report that showed hepatitis B surface protein could be expressed in tobacco and that the antigen was immunologically and chemically identical to its native counterpart (Mason et al., 1992). A few years later, transgenic potato was developed and tobacco expressing *E. coli* heat-labile enterotoxin was shown to be able to immunize and protect mice against the lethal toxin upon oral administration (Haq et al., 1995). Since then, a few dozen antigens have been successfully used as vaccines in different plant systems (Manson et al., 2002). Most of the early reports of plant-based vaccines included antigens that are mucosal in nature, e.g. diarrhea causing organisms and intestinal toxins such as cholera toxin B subunit, Norwalk virus capsid protein, mink enteritis virus, the glycoprotein S from the swine-transmissible gastroenteritis coronavirus, respiratory syncytial virus F protein, spike (S) protein of transmissible gastroenteritis virus, and some others. The approach of expressing mucosal antigens in plants with the ultimate use as oral formulations is justified by the fact that theses antigens are derived from pathogens that typically inhabit mucosal surfaces. However, other antigens not associated with mucosal pathogenicity have been expressed in plant systems and, in some cases, used successfully as oral vaccines that can elicit an immune response and confer protection. Examples include the human hepatitis B virus envelope protein and HIV gp41.

A plant-based immunocontraceptive vaccine for the control of overpopulated mammals poses very desirable advantages over today's methods of control. A transgenic plant useful for controlling free-ranging wildlife can be used in one of two ways (Smith et al., 1997). The genetically modified plant could be spread and allowed to be self-propagating. Creation of male-sterile lines or plants with transgenic chloroplasts (which exhibit maternal inheritance) will enable much more rigorous containment (Mason et al., 2002) and thus reduce concern over free-ranging transgenic plants.

Alternatively, the modified plants can be grown under controlled conditions under the regulatory aspects of plant-derived vaccines (Mason et al., 2002). These plants could be harvested and distributed as bait formulations that can be positioned at strategic places. This approach may significantly increase the cost of production.

### Transgenic Plant With Transient Transgene Expression

One of the major considerations for developing transgenic plants expressing heterologous proteins is the choice of transfection systems. Currently there are at least two distinct methods for producing transgenic plants. These include stable and transient expression (Lessard et al., 2002). Transient expression consists of a recombinant plant virus carrying an antigenic gene. By infecting the plant, the foreign protein is produced either in the entire plant or in specific tissue, depending on the virus and promoter used. In general, protein expression levels are high due to high number of gene copies in each virus and traditionally recovery of the expressed product has been an easy task. The down side of using this method is that expression lasts only as long as the plant is infected and efforts need to be made to keep the infection ongoing. Furthermore, this system suffers from instability and loss of genes that are larger that 1kb (Mason et al., 2002).

Methods of engineering virus particles that can express a peptide (or epitope) have been developed. This has been accomplished by genetically fusing the epitope to virus coat protein, such as the coat protein of tobacco mosaic virus or the cowpea mosaic virus. The desired antigen is expressed on the surface of the virus once the recombinant virus particles undergo self-assembly. When injected, such particles have been shown to elicit protective antibodies to an antigen of mink enteritis virus expressed on the surface of cowpea mosaic virus. This system is limited, however, by the size of the peptide to be introduced since only peptides less than 25 amino acids have been successfully used in this manner.

Although transient transfection may work well for a selected number of vaccination strategies where the antigen is purified and injected, this approach may not be appropriate for oral vaccination strategies.

### Transgenic Plant With Stable Transgene Expression

Stable transgene expression involves the integration of the transgene into the chromosome of either the nucleus or the chloroplast of the plant cell. Once a gene is integrated into a chromosome, it will be propagated by Mendelian inheritance to subsequent generations. The method of stable expression most often used is the *Agrobacterium*-mediated transformation. This method takes advantage of the naturally occurring plant genetic transformation caused by the plant pathogen *Agrobacterium tumefaciens* (Lessard et al., 2002). This bacterium causes a plant disease with symptoms that include the development of plant tumors. During the infection period *Agrobacterium* transfers a segment of its own DNA into the plant chromosomes (Lessard et al., 2002). This is accomplished by the large Ti (tumor-inducing) plasmid. This plasmid contains a segment called the T-DNA that will get integrated into the plant chromosome as a result of infection. The T-DNA is bounded by a 25 bp repeat sequence that encloses genes called the *vir* genes that are important in the virulence of the pathogen. Within the T-DNA there are additional segments that encode gene products that will direct the production of certain amino acids called opines that typically are not products of plant metabolism. Opines constitute vital sources of nitrogen and carbon for the *Agrobacterium.* Additionally, the T-DNA contains genes that direct the production of certain plant hormones called cytokinins that are important in plant ontogeny transformation.

The naturally occurring Ti plasmid is larger than 200 Kb which makes it virtually impossible for routine manipulation. Therefore, plant biotechnologists have substituted most of the genes in the T-DNA plasmid and replaced them with multiple cloning sites and other features employed in current genetic manipulation methods. Such plasmids lack the original genetic composition, being left with only the virulence genes and the T-DNA important for chromosome integration. To improve their manageability, the new vectors contain features that make their manipulation possible not only in *Agrobacterium* but also in *E. coli.* These plasmids are called binary vectors and contain origins of replication that are compatible to both bacterial species. These artificial plasmids also include several features important for expression of the heterologous gene. Perhaps the most critical of them is the promoter that will drive the expression of the antigenic gene. Several considerations should be taken into account in making a choice of promoter to use. There are a number of promoters regulating the spatio-temporal expression of genes. If, for example, the expression is desirable in a specific part of plant such as the fruit, a fruit-specific promoter like the 2A11 from tomato should be used (Van Hareen and Houck, 1993). However, for most applications strong viral promoters that can drive constitutive gene expression are employed. Perhaps the best described and most widely used is the 35S promoter from Cauliflower Mosaic Virus promoter (Benfey et al., 1990). This promoter is characterized by inducing high expression in vascular tissues, but significantly lower expression is found in meristem tissue.

Besides promoters, a plant expression cassette needs to have additional features for successful transgene expression. These include a polyadenylation signal, transcription termination signal, introns, and 5' untranslated leader sequences. These sequences form integral parts of genes and have been spliced into the Ti plasmid for optimum expression.

Another aspect of *Agrobacterium-*mediated transformation is that its production requires genetic selection that differentiates transformed from an overwhelming amount of untransformed plant tissue. For this, genes that endow the transgenic tissue to survive some form of selection are needed within the T-DNA boundaries. The most widely used is the kanamycin resistance gene (Lessard et al., 2002).

In accordance with the present invention there may be employed conventional molecular biology, microbiology, and recombinant DNA techniques within the skill of the art. Such techniques are explained fully in the literature. See e.g., Maniatis, Fritsch & Sambrook, "Molecular Cloning: A Laboratory Manual (1982); "DNA Cloning: A Practical Approach," Volumes I and II (D.N. Glover ed. 1985); "Oligonucleotide Synthesis" (M.J. Gait ed. 1984); "Nucleic Acid Hybridization" [B.D. Hames & S.J. Higgins eds. (1985)]; "Transcription and Translation" [B.D. Hames & S.J. Higgins eds. (1984)]; "Animal Cell Culture" [R.I. Freshney, ed. (1986)]; B. Perbal, "A Practical Guide To Molecular Cloning" (1984).

*Agrobacterium*-mediated transformation has been the method of choice for introducing genes into agriculturally important crops since it is well characterized and the methodology has been widely standardized. However, this approach has presented several problems that have prevented its use for certain applications. Perhaps the biggest drawback is that the protein expression levels have been typically low. On average, the expression levels are around 0.01 % or less of the total soluble protein, depending on the antigen and plant being used (Daniell et al., 2001). Several causes have been recognized for obtaining such a low quantity of desired protein. Perhaps most crucial in certain instances is that the expression machinery has been shown to have considerable preference in its codon usage. Many xenogenic genes contain codons that are used in lower frequencies in plants and therefore, their expression will be low. This can be overcome by making the appropriate adjustments to the sequence via directed mutagenesis such that the codons are compatible.

Another effect that prevents the overexpression of foreign genes is called gene silencing. This could be the effect of repetitive regulatory sequences, integration of multiple copies of the heterologous gene, or even as a result of efficient transcription of transgenes. It can take place both at the transcriptional and posttranscriptional levels (Fagard and Vaucheret, 2000). Lastly, "positional effects" have been known to be a factor in inconsistent expression and even complete expression silencing. This phenomenon occurs as a result of random integration of the T-DNA into nuclear DNA. Transcription of the transgene will be subjected to the same regulatory forces exerted on the surrounding sequences (Fagard and Vaucheret, 2000).

Until the mid-nineties *Agrobacterium* was incapable of being used on monocotyledonous plants, many of which are highly desirable to transform, such as palms and grains. Other methods of plant transformation are now possible. These include techniques that have a more physical basis such as particle electroporation and particle bombardment (colloidal gold coated with plasmid DNA), resembling DNA vaccine gene delivery (Newell, 2000). These biophysical methods have typically resulted in integration of several copies of the heterologous gene, thus enhancing transgene expression levels. In addition, methods have been developed to introduce foreign genes into the chromosome of the chloroplast. Since this organelle exhibits high copy numbers (up to 10,000 per cell), expression of the foreign gene is typically much higher (Daniell et al., 2001). Considering the prokaryotic nature of chloroplasts, effects that deter transgene expression such as gene silencing and positioning effects are less likely to occur.

### Plant Systems

Depending on the reasons behind using plants as vaccines, different plant systems should be considered. *Arabidopsis thaliana* can be used to check whether a gene will be compatible with the plant expression machinery. This model plant has been genetically and biochemically characterized extensively and its short generation time allows production and testing of transgene in a matter of weeks. Extrapolating successful transgene expression to other plant systems may not be adequate for every antigen, but certain patterns or discrepancies such as toxic products or problematic codons could be observed in the *Arabidopsis* model. However, due to its small size and amount of tissue it can yield, vaccination trials may not be appropriate.

Tobacco has been characterized for transgenic plant production and it is one of the preferred systems routinely used in transfection experiments (Mason et al., 2002). Tobacco is capable of providing abundant material for protein characterization and for vaccine trials despite the fact that it takes a few months for its development. It offers the advantage of a simple and efficient transformation system that is highly standardized. However, before any vaccination protocol is implemented, the material needs extensive processing, given that it has to be detoxified due to its high alkaloid content.

More palatable delivery systems are needed. The most widely edible plant used in oral vaccination experiments is potato. This tuber has the advantages of being easily transformed, clonally expandable, and its industrial processing is well established (Mason et al., 2002). Tuber specific promoters have been identified and the tuber can be eaten raw by many species of animals. The downside of this method is that the tuber contains a relatively low protein content and theoretical "potato dosages" may be large. Despite this, it has been reported that oral administration of the raw tubers provides immune responses and protection in mice. Moreover, not only do plants need to deliver high levels of antigens to make a more potent formulation but, in addition, economically feasible plants that can be grown locally (in controlled environments) need to be considered. For this, investigators have contemplated the use of tomatoes, bananas, alfalfa, legumes, and cereals (Mason et al., 2002).

### Immunological Considerations of Plant-Based Vaccines

Plant-based vaccines, as oral antigens, are expected to stimulate mucosal immune responses. The mucosal immune response is characterized by the production of secretory IgA that can provide a line of defense against antigens that find entry into the host through the epithelium. Mammals typically secrete 40 mg of IgA per kilogram of body weight every day. Directing secretory IgA to neutralize certain antigens is a highly desirable strategy that not only can provide protection from pathogens but also can be used in immunocontraception.

An antigen that is orally delivered has to overcome major obstacles to induce an effective immune response. The plant-produced antigen is subjected to hostile stomach environment that consists of very low pH and a number of proteases. Depending on the antigen, it has been estimated that the amount of intact antigen that is absorbed ranges between 0.001% and 1%. The robustness of the plant cell wall can protect the antigen.

Another major difficulty that an antigen has to overcome is immunological tolerance. Millions of potential antigens are being consumed in food. The immune system has devised a number of mechanisms to suppress a response against harmless food antigens while still reacting against potentially harmful pathogens. Diseases such as coeliac and Crohn's disease are believed to be the result of breakdown in oral tolerance to gluten in wheat and to antigens from commensal bacteria. In the plant-based vaccine context, tolerance has been regarded by many as a major obstacle that could hamper widespread development of this technology. However, many investigators have proposed strategies that can be useful in breaking oral tolerance. As in systemic immunization, oral antigens can be delivered in the presence of mucosal adjuvants to elicit an adequate immune response as well as protection. These adjuvants are usually bacterial toxins such as cholera toxin or the *E. coli* heat labile enterotoxins from bacteria that inhabit the mucosal epithelium. Not surprisingly, these antigens were the first to be considered for the early proof-of-principle experiments with plant-based vaccines (Mason et al., 2002). Antigens such as hepatitis B surface antigen expressed in potato have been shown to elicit adequate immune responses and protection upon oral administration in the presence of cholera toxin. The main drawback of oral adjuvants is that they are associated with high toxicity. Methods generally available in the art can be used to provide detoxified forms (toxoids) of these factors that will not loose their adjuvancy.

The present invention is directed to a genetically modified plant that expresses an immunocontraceptive comprising an egg- or sperm-specific polypeptide or antigenic fragment thereof. The sperm-specific polypeptide includes lactate dehydrogenase-C such as rat or murine lactate dehydrogenase-C. Representative polypeptides of sperm-specific lactate dehydrogenase-C include amino acids 5-17, 44-58, 61-77, 97-110, 180-210, 211-220, 231-243, 283-306, 307-316, 101-115 of murine lactate dehydrogenase-C. These contraceptive agents are useful in controlling the size of an animal population by inducing sterility in animals that have ingested the genetically-engineered plant materials. In general, the plant materials include potato, tobacco, rice, bananas, wheat, corn, tomato, any fruit, vegetable, or legume.

In one embodiment of the present invention, there are provided successfully constructed *Arabidopsis* and tobacco strains expressing mouse lactate dehydrogenase-C up to 0.01% of total soluble protein. This recombinant protein showed comparable biochemical and immunological properties to its native counterpart and to the *E. coli* overexpressed protein. Extracts prepared from these plants were fed to mice and in preliminary studies most animals produced lactate dehydrogenase-C specific antibodies.

The present invention is also directed to methods of using these contraceptive agents to decrease the fertility of an animal. These contraceptives are first dispersed as baits in the habitats of the targeted animals, and ingestion of these baits by the animals would induce sterility in those animals. Susceptible animals include mice, rats, deer, elephants, water buffalo, feral horses, foxes, urban or wild dogs, urban or wild cats, rabbits, and other potentially overpopulated species causing economic damage to society.

The following examples are given for the purpose of illustrating various embodiments of the invention and are not meant to limit the present invention in any fashion. The present examples, along with the methods, procedures, treatments, molecules, and specific compounds described herein are presently representative of preferred embodiments. One skilled in the art will appreciate readily that the present invention is well adapted to carry out the objects and obtain the ends and advantages mentioned, as well as those objects, ends and advantages inherent herein. Changes therein and other uses which are encompassed within the spirit of the invention as defined by the scope of the claims will occur to those skilled in the art.

### EXAMPLE 1

### Cloning of Plasmid Containing Full Length Lactate Dehydrogenase-C cDNA

For the development of transgenic plants via *Agrobacterium-*mediated transformation the antigenic genes need to be cloned into a modified form of the Ti plasmid from *Agrobacterium tumefaciens* constructed by Schardl *et al*. (1987) with the purpose of simplifying the expression of foreign genes in plants. The plasmid was further engineered to contain EcoRI and HindIII sites and was called pLBJ21 (Figure 2). This pLBJ21 plasmid was used to construct a plasmid containing a full length lactate dehydrogenase-C cDNA as described below.

Plasmid pDNA3.1-LDH-C was provided by Dr. Erwin Goldberg from Northwestern University, Evanston, Illinois. This plasmid contains the cDNA of mice lactate dehydrogenase-C (LDH-C) between two EcoRI sites (Figure 3). The plasmid was transformed into XL-1 blue electrocompetent cells (Stratagene). The plasmid was isolated using the Quiaprep Spin Plasmid kit (Quiagen) following the manufacturer's instructions. The isolated plasmid was subjected to restriction digest with EcoRI and the lactate dehydrogenase-C sequence was recovered by agarose gel purification. The lactate dehydrogenase-C sequence was then cloned into the plasmid pLBJ21.

To prepare the plasmid pLBJ21 for cloning, the plasmid was transformed into DH5α cells (Novagen). A 10 ml LB culture containing 15 µl of tetracycline (5 mg/ml) was inoculated and grown with aeration at 37°C overnight. The plasmid was isolated using the Quiaprep Spin Plasmid kit (Quiagen) following the manufacturer's instructions. Since pLBJ21 is a low copy plasmid, a 10 ml LB culture had to be used. The plasmid was subjected to restriction endonuclease digestion with EcoRI and was used as a cloning vector.

After a plate with ~100 colonies was obtained from the cloning procedure, the cells were PCR screened for vectors containing the insert in the correct orientation. Since only one restriction enzyme was used, the gene could be inserted in the reverse direction. The colonies were PCR screened using the upstream primer LMV355Promoter (5'AGGACACGTGAAATCACCA) (SEQ ID No. 1) which is complementary to the Cauliflower Mosaic Virus Promoter and anneals to the vector. As a downstream primer the LDH-C3' was used (5'NNNNNGGATCCTACTATA ACTGCACATCCTTCTG) (SEQ ID No. 2). The new plasmid was called pLBJ21-LDH-C (Figure 4). The suspected positive colonies were used to inoculate a 10 ml LB culture with 15 µl of tetracycline (5 mg/ml) and the plasmid was isolated using the Qiaprep Spin Plasmid kit (Qiagen). The plasmids were submitted for sequencing to confirm the correct lactate dehydrogenase-C sequence was contained in pLBJ21-LDH-C.

### EXAMPLE 2

### Cloning of Plasmid Containing A Peptide Epitope of Lactate Dehydrogenase-C

Plasmid pcDNA3.1-SPV was designed to express an immunodominant lactate dehydrogenase-C epitope. The synthetically constructed minigene SPV (Short Peptide Vaccine) includes amino acids 5-15 of lactate dehydrogenase-C coupled via a short spacer to a promiscuous T-cell epitope from tetanus toxin. The SPV was first constructed by a multi-step PCR using long oligonucleotides, and then the gene was cloned into appropriate vector. In this case, the synthetic gene was subcloned in a series of steps to create a peptide vaccine vector (see Figure 5).

In the first step, a plasmid with the intact SPV region was used as a template for PCR so that the product would be comprised solely of the SPV sequence. As stated before, the sequence of this minigene includes amino acids 5-15 of lactate dehydrogenase-C (LDH-C). It does not contain a start ATG codon. By using PCR, the first 5 amino acids of LDH-C (amino acid 1 being an ATG) could be included in the sequence of SPV. This new minigene included amino acids 1-15 of LDH-C, making it a more "natural" peptide vaccine in addition to including a start codon. The PCR product was cloned into the pCR2.1 (Invitrogen, Ca) TA vector. This vector is conveniently designed for cloning PCR products easily without purification. Small fragments such as the SPV can be conveniently cloned and the complete procedure will take 2-3 days. The decision to use pCR2.1 in this instance was made because many researchers have encountered numerous difficulties trying to clone PCR products, in particular when these are as small as is the case of SPV. A clone from the pCR2.1 ligation reaction was sequenced with the purpose of confirming its integrity and then it was submitted to restriction digestion with EcoRI. The product from the digestion was eventually inserted into pcDNA3.1 to make pcDNA3.1-SPV.

The cloning of the SPV into the plant expression vector pLBJ21 was similar to the cloning of pLBJ21-LDH-C described above. Plasmid pcDNA3.1-SPV was subjected to restriction digest using EcoRI. After digestion, the gene was cloned into digested pLBJ21. Successful cloning was confirmed by PCR screening. The new plasmid was called pLBJ21-SPV (Figure 6). A few colonies from positive cloning were selected and a plasmid preparation was done using the Qiaprep Spin Plasmid kit. The plasmids were subjected to sequencing using the CaMV primer described above.

### EXAMPLE 3

### Transformation of Plasmids Into Agrobacterium tumefaciens

Strain GV3101(pMP90) of *Agrobacterium* (Knocz and Schell, 1986) was frozen in LB media supplemented with 10% glycerol. From this glycerol stock a 5 ml culture was inoculated and grown overnight in a 30°C incubator shaking at 200 rpms. A dense culture was obtained next morning and was used to make a stock of electrocompetent *Agrobacterium.* One µl of each plasmid (approximately 50 ng) was mixed with 20 µl of freshly prepared cells and the mixture was incubated on ice for 30 minutes. The cells were transferred to an electroporation chamber Micro Electro Chamber (Gibco BRL Life Technologies). The chambers were placed in the Cell Porator Electroporation System I (Gibco BRL Life Technologies) and the transformation was performed according to the manufacturer's instructions.

The transformed *Agrobacterium* were recovered in 250 µl of SOC media at 30°C while shaking at 200 rpm for 1 hour. The cells were further plated in double selection LB agar plates containing 50 mg/L kanamycin and gentamycin. The plates were incubated at 30°C for 48 hours. About 10 colonies from each plate were PCR-screened. PCR was performed in a Perking Elmer Gene Amp PCR system 2400 using the following temperature sequence: 5 minutes, 94°C; 30 cycles of 45 seconds, 94°C, 45 seconds, 55°C, 1.5 minutes, 72°C; 10 minutes, 72°C. After PCR, positive clones were visualized on agarose gel. It is important to run a positive control (the original insert) and a negative control (water) so bands in the gel can be compared accordingly. Successful transformations were kept at -80°C in glycerol stocks until further use.

### EXAMPLE 4

### Development Of Transgenic Arabidopsis Thaliana By The Vacuum Infiltration Technique

Adult *Arabidopsis* were transfected with the modified *Agrobacterium* by applying a vacuum forcing transfection as described below. During transfection, the T-DNA plasmid was incorporated into the chromosome of the plant. The progeny of these plants will be transgenic and can be selected using an appropriate antibiotic.

On the first day of the experiment about 25 seeds from the laboratory wild type strains Wassilewskija (WSwt) and Landsberg *erecta* (Le*r*wt) of *Arabidopsis thaliana* were sprinkled in 12 small pots containing autoclaved soil that was previously moist with tap water. The planted seeds were further soaked with tap water and were covered with a plastic lid. The pots were covered from light and were placed at 4°C for 2 days. This ensured all the plants would germinate uniformly.

On the 3^{rd} day the pots were moved to a 20-22°C incubator with a constant 24-hour exposure to light. The lid was not removed for 3-4 days or until the seeds were fully germinated. Small leaves and stems were visible at this time. The plants were kept in this incubator for the rest of the experiment and were watered and fertilized regularly. Approximately 4-5 weeks after planting, the adult plants were ready for transfection. The primary inflorescence should be 5-15 cm long and the secondary inflorescences should be appearing at the rosette.

A 125 ml LB culture containing 50 mg/L kanamycin and gentamycin was inoculated with previously prepared glycerol stocks of *Agrobacterium* and grown for 2 days at 30°C with constant shaking at 200 rpm. The cultures were harvested by centrifugation at 500 g at 4°C for 10 minutes and the supernatant was discarded. The bacteria were resuspended in 250 ml of infiltration medium composed of 2.2 g Murashige and Skoog salts (Sigma), 1 X B5 vitamins (Sigma), 50 g sucrose (Sigma), 0.5 MES (Sigma) pH 5.7-5.8, 0.044 µM benzylaminopurine (Sigma), and 200 µl of the surfactant Silwet L-77 (Lehle Seeds) per liter (Bechtold and Pelletier, 1998). A 1000X stock of B5 vitamins consists of 1 g myoinositol, 0.1 g thiamine HCl, 10 mg nicotinic acid, and 10 ml pyridoxine HCl per liter.

The 250 ml infiltration medium with the *Agrobacterium* was transferred to a plastic 250 ml beaker in a vacuum dome. The plants were submerged in the medium upside down being careful not to introduce soil from the pots. The vacuum dome was sealed and a vacuum was created using an electric vacuum pump for 15 minutes. After infiltration, the plants were dripped to remove extra medium and kept in a moist environment for a day by replacing the plastic lid. The treated *Arabidopsis* were handled as previously described until the plants were seeding. At this point, watering was stopped and the seeds were collected from the dried plants. The seeds were dried in a dessicator for 3 days.

The seeds were ready for selection at this point and they were sterilized by placing them in a folded piece of filter paper closed accordingly to make a "tea bag". The filter paper was submerged in a solution of 20% bleach with 0.1% tween 20 (Sigma) for 15 minutes. They were washed afterwards 3 times with distilled water. After the seeds were sterilized they were placed and distributed evenly in GM plates containing 100mg/L of kanamycin (Sigma) and timetin (SmithKline Beecham) under sterile conditions. The GM plates were composed of 0.5 X Murashige and Skoog salt mixture (Sigma), 1% sucrose, MES pH 5.7 (Sigma), and 0.8% tissue culture grade agar (Sigma). The plates were kept sealed with Parafilm at 4°C in the dark for 2 days. On the third day, the plates were moved to an incubator at 22°C with a 24-hour light cycle.

The plates were incubated for 7-10 days until the seeds that were transformed germinated. Untransformed plants germinated but their development was stopped by kanamycin. The small plants were transferred to pots containing autoclaved, moist soil. Up to 30 plants were planted in each pot. The pots were covered with a transparent lid for an additional 3 days after which the transgenic plants were grown normally under the described conditions.

### EXAMPLE 5

### Development of Transgenic Tobacco

This method was adapted from the protocol developed by Horsch et al. (1985). It used a plant tissue culture format to transform explants. The strain of *Nicotiana tabaccum* Xanti N'N' was used for transfection. The seeds were sterilized as described previously in the *Arabiodpsis* method. They were further plated on MS104 agar under sterile conditions and kept in an incubator at 22°C with constant 24-hour light. MS 104 plates consist of 2.2g Murashige and Skoog's salts (Sigma), 3% sucrose, 0.5g MES pH 5.7, 0.1 g/L naphthalene acetic acid (Sigma), 1.0 g/L benzyladenine (Sigma), and 0.8% tissue culture grade agar. The seeds germinated for about 2 weeks until the plants grew shoots that were approximately 1cm long. At this point the plants were ready for transfection. The explants were prepared by excising the cotyledon from the plants leaving behind the hypocotyl and the outer portions of the leaf. The pieces of explants, excluding the cotyledon, were placed in fresh MS 104 agar plates assuring that each piece of explant was in contact with the medium.

An *Agrobacterium* culture was prepared the day before transfection. For this, a 3ml LB culture supplemented with 50 mg/L of kanamycin and gentamycin was inoculated with *Agrobacterium* from a frozen glycerol stock. The cultures were placed in a shaker incubator at 30°C spinning at 200 rpm overnight. A turbid culture was recovered and 100µl of it were added to 3ml of liquid MS 104 medium. The *Agrobacterium* mixture was added to the plates containing the explants ensuring that the liquid covered the explants in their entirety. The excess liquid was removed by aspiration, the plates were sealed, and they were placed in an incubator as described above. After 72 hours, the transfected explants were moved under sterile conditions to fresh MS 104 agar plates containing 100 mg/L kanamycin and 100 mg/L timetin. Under these conditions the transformants grew into undifferentiated tissue forming tumor. After two weeks visible stems and leaves developed from each tumor. The plant tumors were moved to MS 104 agar plates without hormones (naphthalene acetic acid and benzyladenine). This promoted the formation of roots. The plants were incubated for about a week until roots were formed. The transformants were then moved individually to pots containing moist soil under a humid atmosphere. This was accomplished by placing a transparent lid covering the pots. After 4-6 days, the plants started to adapt to their new soil environment and the lid was removed. The plants were further housed indefinitely at the University of Texas of Austin greenhouse facility where they were watered and fertilized regularly.

### EXAMPLE 6

### Expression of Lactate Dehydrogenase-C in Arabidopsis

The expression of mouse lactate dehydrogenase-C (LDH-C) by transgenic *Arabidopsis* was tested using immunoblots. This required extraction of protein from the plants and polyacrylamide gel electrophoresis (PAGE). The protein preparation was performed using a protocol developed by Tanaka and Hurkman (1986). About 3 grams of plant material (leaves, stems, and roots) were frozen at -80°C and ground with a mortar and pestle in the presence of liquid nitrogen until the plants were pulverized. At this point, 2ml of extraction buffer (0.7 M sucrose, 0.5 M Tris, 30 mM HCl, 0.1 KCl, 2% β-mercaptoethanol, 10 mM phenylmethylsulfonyl fluoride (PMSF, Sigma), and 10X Complete Protease Inhibitor Cocktail (Roche)) were added and the grinding continued. The pulp was transferred to a Corex centrifuge tube and an additional 6ml of extraction buffer was added. The tubes were incubated for 30 minutes at 4°C and later centrifuged at 5,000 rpm for 10 minutes. The supernatant was transferred to a fresh Corex tube and was vigorously mixed with an equal volume of water-saturated, ice-cold phenol. The mixture was incubated for 45 minutes at 4°C. Phases were separated by centrifugation at 6,000 rpm for 10 minutes. The phenol phase and the interphase were transferred to a third Corex tube and 5 volumes of 0.1 M ammonium acetate in ice-cold methanol were added to it. Proteins were precipitated overnight at -20°C and further isolated by centrifugation at 5,000 rpm for 10 minutes. The pellet was air-dried and was further resuspended in phosphate buffered saline (PBS) for further testing.

Thirty µl of the extracts were diluted in 4X PAGE loading buffer, boiled for 5 minutes, and loaded on a 10% polyacrymalide gel. After the gel was finished, the proteins were transferred to a polyvinylidenefluoride (PVDF) membrane (Pierce). For Western Blot analysis, the primary antibody was an affinity purified rabbit anti-mouse lactate dehydrogenase-C (provided by Dr. Erwin Goldberg) used at 1:10,000 dilution. For the secondary antibody, a commercial grade goat anti-rabbit IgG-HRP (Kirkegaard and Perry Laboratories) was used at the recommended 1:10,000 dilution.

Figure 7 shows a western blot of plants from pots belonging to transformants 1, 4, and 11 as well as wild type plants and mouse testis as control. The transgenic plants from clones 1 and 4 expressed high levels of lactate dehydrogenase-C. This is evident since the signal matches the band seen in the mouse testis lane. The cause for the lack of expression of clone 11 may be due to gene silencing mutation. In order to rule out lack of detection due to insufficient protein loading, the same membrane was stripped and re-tested with a polyclonal rabbit antibody against the *Arabidopsis* novel cap binding protein (eIF-4E), which is constitutively expressed. Enough protein was loaded since eIF-4E can be clearly detected in every *Arabidopsis* sample but not in the mouse testis extract.

Testing the expression of mouse lactate dehydrogenase-C in *Arabidopsis* was extremely useful in that it showed that this enzyme does not inflict a detrimental effect in plants. Previously, a concern was raised that this glycolytic enzyme would interfere with plant metabolism and therefore prevent normal plant development or induce gene silencing.

### EXAMPLE 7

### Expression of Lactate Dehydrogenase-C in Tobacco

Protein expression can be examined by western blot as described above. A second method used to corroborate the expression of lactate dehydrogenase-C in tobacco was to measure its enzymatic activity. The activity would not only show successful protein expression but also would provide evidence that the plant-produced enzyme is structurally and, most likely, immunologically viable. This method is an adaptation of an original protocol developed by Babson and Babson (1973). The method detected protein expression and activity using a colorimetric assay in which the reduction of NAD is coupled to the reduction of a tetrazolium salt providing a red precipitate. The assay was performed in a polyacrylamide gel electrophoresis system using non-denaturing gels. A 10% native polyacrylamide gel was prepared, and no SDS was used in any of the buffers nor an oxidizing agent like dithiothreitol was used. The protein samples were not boiled and precautions were taken to ensure the proteins were native and active.

Protein extracts were prepared as those for lactate dehydrogenase-C expressing plants. The extracts were mixed with 4X native loading buffer and loaded in a discontinuous Tricine-sodium dodecyl sulfate-PAGE designed for optimal peptide separation by Schagger and von Jagow (1987). The samples were run at 125 volts with constant amperage for 3 hour. The gel was washed once with deionized water and then submerged into 20 ml of substrate solution consisted of 50 mM Tris-HCl pH 8.2 and 50 mM L(+) lactic acid solution (Miles Chemical Co., Clifton, NJ. The gel was incubated for a few minutes at 37°C and then color reagent 40 mg of INT (2-p-iodophenyl-3-p nitrophenyl-5-phenyl tetrazolium chloride) (Sigma), 100 mg of NAD (Sigma) and 10 mg of PMS (phenazine methosulfate) (Sigma) in 20 ml of distilled water was added.

An example of native gel staining with tetrazolium salt was shown in Figure 8. The results of this gel indicate that plants from clone 1 and clone 4 expressed lactate dehydrogenase-C, since they matched the band of the mouse testis in activity and migration. The recombinant form of lactate dehydrogenase-C (rLDH-C) in lanes 2 and 3 migrated much further in the native gel. This is because the rLDH-C contained a histidine-tag that, under the conditions to which the gel is subjected, it contributes significantly to the overall negative charge of this protein.

### EXAMPLE 8

### Quantification of Lactate Dehydrogenase-C Expression In Tobacco

Once the expression of lactate dehydrogenase-C was established, it was important to quantify how much protein the tobacco could produce such that known amounts of protein can be administered to the mice. A sandwich ELISA was developed to quantify the amount of lactate dehydrogenase-C expressed in the transgenic tobacco plants. Here, a capture antibody was first plated in the microwells and then the extract was added. The plates were washed such that only the antigen bound to the antibody was left in the well. Then the antigen was tested with a secondary antibody (in this case a biotinylated form of the same polyclonal antibody).

### Tobacco Extract Preparation

A small sample of transgenic or wild type tobacco leaf tissue (~100 mg) was crushed with a mortar and pestle in the presence of liquid nitrogen. The pulverized tobacco material was transferred to a 1 ml glass tissue grinder (Corning) at 4°C. Immediately thereafter, 500 µl of pre-chilled extraction buffer were added (PBS supplemented with 2 mM PMSF, 2X Complete Protease Inhibitor Cocktail (Roche Biochemicals) and 2 mM EDTA). The tobacco was ground until a homogenous solution was obtained. The solution was transferred to a 2 ml microtube and was centrifuged in an Eppendorf Centrifuge 5417C (Eppendorf) at maximum speed for 20 minutes at 4°C. The supernatant was transferred to a fresh tube and the protein concentration was determined by the Warburg-Christian method. All samples to be tested including the wild type sample were normalized to a common protein concentration by dilution with phosphate buffered saline (PBS) such that accurate measurement of lactate dehydrogenase-C (LDH-C) could be performed.

### Sandwich ELISA Assay

Purified polyclonal antibody against lactate dehydrogenase-C provided by Dr. Erwin Goldberg (Northwestern University) was used as the capture antibody. One hundred ng of the antibody were added to a microwell in a 96-well format in a total of 50 µl of capture buffer (100 mM carbonate buffer, pH 9.5). The ELISA plate was incubated at 4°C, washed 3 times with wash buffer (PBST) and extracts from transgenic or wild type plants were added in different concentrations. The plates were incubated for exactly one hour at room temperature and subsequently washed as describe above. Fifty µl of a 1:10,000 dilution of 1 mg/ml biotinyalated rabbit anti-mouse lactate dehydrogenase-C were added and the plates were incubated for one hour. The plates were washed as above and were then incubated for 30 minutes with 50µl of horseradish peroxidase-neutravidin (Pierce) (1mg/ml) diluted 1:400 with blocking buffer. The plates were submitted to a last wash and then 100 µl of the substrate ABTS (Moss Inc., Pasadena, MD) was added. The plates were kept in the dark for 20 minutes and the developing reaction was stopped with 0.5 M oxalic acid (Sigma). The plates were read in an ELISA spectrophotometer at 414 nm.

Data from the ELISA assays indicated that the tobacco extract contained up to 300 ng/ml of lactate dehydrogenase-C. The total protein concentration of each extract was quantified and they turn out to be approximately 4 to 5 mg/ml consistently, including the wild type tobacco extract. This means that tobacco lactate dehydrogenase-C production is about 0.01 % of the total soluble protein. This value is comparable to other antigens that have been expressed and successfully used in tobacco (Daniell et al., 2001).

### EXAMPLE 9

### Preparation of Tobacco Protein Extract for Vaccine Administration

For vaccine trials, tobacco extracts were prepared and partially purified. Possible toxic alkaloids contained in tobacco were detoxified and the lactate dehydrogenase-C in the extract was concentrated so that the administered vaccine volume was not too great to cause animal discomfort.

One hundred grams of leaves were collected from wild type plants or plants expressing greater levels of lactate dehydrogenase-C. The leaves were crushed to a fine powder with a mortar and pestle in the presence of liquid nitrogen. The pulverized tobacco was transferred to a blender and mixed with 250 ml of ice-cold extraction buffer consisted of 2X PBS supplemented with 2 tablets of Complete Protease Inhibitors (Roche Biochemicals), 2mM PMSF, 2 mM EDTA, 2 mM DTT (Sigma), and 2 mM β-2ME (Sigma). The powder was blended to homogeneity at 4°C. The homogenate was filtered using cheese cloth and was then collected in 50 ml centrifuge tubes. The tubes were centrifuged for 30 minutes at 40,000 g. The supernatant was collected and its volume was measured. It was brought to 40% saturation with enough crushed solid ammonium sulfate (AS) at 4°C with constant stirring. After an hour of precipitation, the suspension was transferred to centrifuge tubes and was centrifuged for 20 minutes at 40,000 g. The pellet was discarded and the supernatant was brought from 40 to 80% saturation with crushed ammonium sulfate. Protein precipitation was performed overnight with constant stirring at 4°C. The centrifugation process was carried out as described above on the next day. The pellet was resuspended in a minimal volume (~10 ml) of PBS. The solution was transferred to a SnakeSkin™ 10,000 MWCO dialysis tubing (Pierce Chemical Co. Rockford, IL) and was dialyzed overnight in PBS with the buffer changed several times.

The next morning, the dialyzed 40-80 AS fraction was placed in a pre-set ultrafiltration apparatus containing a Diaflo membrane XM300 (Amicon, Beverly, MA). The sample was concentrated to about 5 ml. The extract was adjusted to 5% sodium bicarbonate and a few mg of sucrose was added to sweeten the plant extract thereby making it more palatable. The protein concentration was quantified and normalized to the wild type concentration such that equal amounts of protein would be given in extracts from transgenic plants or controls. The samples were aliquoted into small test tubes and stored at -80°C until further use. The amount of lactate dehydrogenase-C contained in each extract was quantified using the sandwich ELISA developed as described in the previous section.

Several dozen mls of partially purified tobacco extract containing 0.28 mg/ml of lactate dehydrogenase-C were prepared. Ten animals received 150 µl of this extract, that corresponded to about 40 µg of lactate dehydrogenase-C. In addition, the formulation was supplemented with 10 µg of the mucosal adjuvant cholera toxin (Sigma), 5% of sodium bicarbonate that neutralized the acid environment of the stomach, and a pinch of sucrose to make the formulation more palatable. As a negative control, an extract from the wild type, prepared using exactly the same protocol, was given to 5 animals (150 µl). As a positive control, 40 µg of pure recombinant lactate dehydrogenase-C (produced in bacteria) was brought to 150 µl in PBS and was administered to 5 animals. Lastly, 5 animals were given half the tobacco dose or 20 µg of lactate dehydrogenase-C in 75 µl of tobacco extract.

To administer the vaccine the sample was loaded into a 1 ml syringe with a stainless-steal animal feeding blunt end needle (Popper & Sons, Inc., New Hyde Park, NY) and the liquid was slowly dispensed into the back of the animal's throat. For two hours prior and 30 minutes after each vaccine administration the animals were deprived of water or food.

### EXAMPLE 10

### Immune Response To Oral Immunization of Tobacco Extracts

Production of antibodies against mouse lactate dehydrogenase-C was monitored periodically by tail bleeding and serum extraction or by collecting vaginal washes as described below.

### Sample Preparation

To prepare the serum, the mice were first restrained, their tail cut at the tip, and a few (7-10) drops of blood were collected in a tube for each animal. The blood was incubated for 20 minutes at 37°C and then at 4°C overnight to complete the clotting process. The blood was spun down in a microcentrifuge at 10000 rpm for 10 minutes. The serum was carefully removed, transferred into a fresh tube, and was either immediately used or kept frozen at -80°C.

Vaginal washes were performed by inserting a blunt pipette tip containing 50 µl of phosphate buffer saltine into the vagina and moving the liquid up and down 10 times. The wash was transferred into a microtube and was kept immediately at 4°C and stored under the same conditions overnight to allow the large particles sediment to the bottom of the tube.

### ELISA Assay

Production of specific antibodies against lactate dehydrogenase-C in serum and vaginal washes was assessed by indirect ELISA. Briefly, 50 µl of 0.01 mg/ml recombinant lactate dehydrogenase-C in capture buffer (100 mM sodium bicarbonate, pH 9.2) was coated onto 96-wells microtiter plate (Flow Laboratories, McLean VA) and were further incubated overnight at 4°C. The plates were washed 4 times with PBST (phosphate buffer saline, pH 7.4, 0.5% Tween 20 (Sigma)), incubated at room temperature in blocking buffer (PBST with 1% protease-free BSA (Roche Biochemicals)) for 30 minutes and washed again once. Serum or vaginal washes were added to the wells up to 50 µl in blocking buffer and the plates were incubated for 1 hour at room temperature. When IgA was tested both serum and vaginal washes were diluted 1:10. When IgG was tested the serum was diluted 1:500 and vaginal washes were diluted 1:10. The plates were washed 3 times as described above and then a secondary affinity purified goat anti- mouse IgG or goat anti-mouse IgA coupled to a horseradish peroxidase was added. Both antibodies were obtained from Kirkegaard & Perry Laboratories (Gaithersburg, MD). For IgG assessment, the antibodies were used at 1:10,000 dilution in blocking buffer. For IgA, a 1:1000 dilution was used. After one hour incubation at room temperature the plates were washed 4 times and 100 µl of hydrogen peroxide-2,2'-Azino-bis-(3-ethylbenzthiazoline-6-sulfonic acid) (ABTS) from Moss Inc. (Pasadena, MD) was added and incubated for 30 minutes in the dark. The reaction was quenched with 100 µl of 0.5 M oxalic acid. The plates were immediately read at 414 nm in an EL340 Automated Microplate Reader (Bio-Tek Instruments Inc., Winooski VM).

### Results

The vaccine was orally administered to female mice 6 times in weekly doses and as a last boost on day 78. Specific serum IgG and IgA and vaginal IgA was monitored periodically on days 1, 15, 29, 38, 45, and 87. Figure 9 shows the production of serum IgG throughout the period of vaccine trials. It is evident that the group orally inoculated with recombinant lactate dehydrogenase-C showed a robust immune response. The groups that were vaccinated with the tobacco extracts showed a modest response after the last boost as compared to the negative controls.

The reason why the recombinant lactate dehydrogenase-C group showed such a robust immune response while the same dose of tobacco extract did not is unknown. It has been hypothesized that since the extract contains a number of potential antigenic proteins from tobacco (possibly more antigenic than lactate dehydrogenase-C itself and in larger quantities), the immune response was diverted from making lactate dehydrogenase-C specific antibodies. This was later confirmed using the indirect ELISAs that were originally designed to detect lactate dehydrogenase-C-specific IgG and which were modified to detect responses to tobacco extracts. The readings from this ELISA were extremely high confirming that the mice developed a strong immune response but it was not, for the most part, directed against lactate dehydrogenase-C.

### EXAMPLE 11

### Purification of Lactate Dehydrogenase-C From Tobacco Leaves

The antibody response against lactate dehydrogenase-C orally delivered using transgenic tobacco extracts was low. It was then decided that testing the immunological properties of the tobacco-produced lactate dehydrogenase-C by other methods would be more appropriate. For this, mouse lactate dehydrogenase-C was purified form tobacco leaves and immune response and infertility were tested by injecting the enzyme in the presence of a commercial adjuvant. This experiment proved the principle that a plant-produced lactate dehydrogenase-C can serve as an effective contraceptive antigen and set the groundwork for future experiments using other systems.

To purify lactate dehydrogenase-C from transgenic tobacco leaves two intrinsic biochemical properties were exploited to simplify this task. Mouse lactate dehydrogenase-C has the property of being both thermostable and an enzyme. Thermostability allows lactate dehydrogenase-C to remain soluble at temperatures where a large number of proteins and other macromolecules in an extract would denature and precipitate. Simply by heating the extracts, a great deal of unwanted contamination was removed. Secondly, since lactate dehydrogenase-C is an enzyme, it has specific affinity for certain molecules, which makes this antigen an excellent candidate for affinity chromatography.

Four hundred grams of tobacco leaf tissues were freshly harvested right before extraction. Leaves were homogenized in a commercial juicer at 4°C and from this moment the sample was always kept at this temperature, unless stated otherwise. The pulp was juiced at least twice and then it was discarded. The homogenate was supplemented with 10X extraction buffer [100 mM phosphate buffer pH 7.0, 10 mM ethyldiaminetetraacetic acid (EDTA), 10 mM dithiothreitol (DTT), 10 mM β-mercaptoethanol (BME), 10 mM phenyl methyl sulfonoflouride, 2 tablets/30 ml of Complete Protease Inhibitor Cocktail® (Roche Biochemical, Germany)]. The sample was centrifuged for 30 minutes at 18,000 rpm in a Sorvall SS-34 rotor (Du Pont, Wilmington, DL). The sample was filtered through a Whatman 158mm filter (Whatman, England) and then transferred to a centrifuge tube.

Mouse lactate dehydrogenase-C is thermostable and this property was exploited for purification purposes. The sample was warmed to 65°C for 45 minutes. The activity of the sample was monitored every 5-10 minutes using a protocol adapted from the Worthington Enzyme Manual (Worthington Biochemical Corporation (1972), Freehold, New Jersey, 7). Lactate dehydrogenase activities were examined by testing the rate of oxidation of NADH to NAD⁺. NADH absorbs light in the 340 nm region of the spectrum whereas NAD⁺ does not. This principle is exploited to quantify dehydrogenase activities.

The sample was cooled back to 4°C and further centrifuged for 15 minutes under the conditions described above. The supernatant was diluted several times in buffer P (phosphate buffer pH 7.0 supplemented with 1 mM EDTA and 1 mM DTT) and it was dialyzed overnight. The next day, it was loaded onto a 15 ml Cibacron Blue® dye chromatography resin column (Bio-Rad Laboratories, Hercules, CA) pre-equilibrated in buffer P. The column was loaded at a rate of 1 ml/minute overnight (the column can be loaded at a faster pace if desired). The column was washed with P buffer until the OD₂₈₀ was below 0.01. Lactate dehydrogenase-C was eluted with about 50 ml of 1mM NADH (Sigma, St. Louis, MO) in 10 mM phosphate buffer pH 7.0. Elution was quantified by testing the lactate dehydrogenase-C activity as described above. NADH was removed from the sample by several rounds of ultrafiltration in a 50 ml concentrator (Millipore, Ireland) with a YM-10 membrane (Millipore). Ultrafiltration was performed until the OD₂₆₀ of the liquid being discarded is 0.01 or less. Ultrafiltration was necessary since the affinity of lactate dehydrogenase-C for NADH is very strong and simple dialysis would not remove the nucleotide.

The sample was concentrated to about 20 ml and was loaded onto a 5 ml 5'AMP-agarose column (product A-3019, Sigma). The column was washed with several dozen mls of buffer P until the OD₂₈₀ was less then 0.01. Lactate dehydrogenase-C was selectively eluted from the column with ~20 ml of a NAD+-pyruvate adduct. The adduct consisted of 50 mg of NAD⁺ (Sigma) and 77 mg of pyruvate (Sigma) dissolved in 100 ml of phosphate buffer, pH 7.0. The purified lactate dehydrogenase-C was subjected to ultrafiltration as explained above to remove the NAD⁺. At this time the solution was concentrated to a minimum volume of about 2 ml. The sample was kept at -80°C until used.

### EXAMPLE 12

### Immune Responses To Lactate Dehydrogenase-C Purified From Tobacco Leaves

Once enough quantities of lactate dehydrogenase-C were purified from plant material, the vaccine trials were performed. Six C57BL mice were used for this experiment. The choice of strain was based on fertility. This strain is very fertile having almost a dozen pups per litter. Forty µg of lactate dehydrogenase-C was emulsified in Titermax Gold® Adjuvant (Sigma, St. Luis, MO) and the formulation was injected into the intraperitoneal cavity of each mouse. As controls, Titermax Gold alone was emulsified in equal amounts of buffer and was injected into 4 mice. Three biweekly boosts of 20 µg lactate dehydrogenase-C emulsified in adjuvant followed until a substantial immune response was obtained. The animals were then mated and their fertility was tested. The immune response was tested on days -1, 6, 12, 19, 26, and 40. IgG and IgA isotypes were tested in serum and vaginal washes. The animals were mated on day 43 where several females were introduced into a cage containing a male previously assessed as fertile. The females were removed and placed in their cage after a clear vaginal plug was detected. Nineteen days after the first animal was impregnated, they were sacrificed and the number of fetuses per animal was counted.

Figure 10 shows the IgG response throughout the vaccination period. From this graph it is evident that a strong response was produced by the tobacco-produced lactate dehydrogenase-C. After the second boost, the mice produced a strong systemic IgG response but this was not significantly increased by a further boost. Since the antigen was injected, a dominant IgG response was expected.

The serum IgA response was also measured although this vaccination strategy was not intended for the stimulation of this isotype. As shown in Figure 11, the mice also produced IgA specific for lactate dehydrogenase-C although it was a more moderate response compared to that given by IgG. As with the IgG response the last two bleedings showed the highest response.

Vaginal IgA and IgG were also tested in washes performed on the same days that the animals were bled. It was found that lactate dehydrogenase-C specific IgA was not present in the vaginal area. This was expected since the vaccine was administered by injection. Lastly, vaginal IgG level was measured for this experiment (Figure 12). As opposed to other vaccine trials where an IgA-based mucosal immune response was expected, IgG was the predominant isotype in this case. The ability to find IgG in vaginal secretions was essential for the success of this experiment. Overall, it can be concluded that the mice were producing primarily an IgG response after injection with lactate dehydrogenase-C purified from tobacco.

### EXAMPLE 13

### Fertility Trials

Data presented above indicated that the animals' immune system responded to lactate dehydrogenase-C immunization by developing lactate dehydrogenase-C specific antibodies. The ability of these antibodies to block fertilization was tested. This was done by mating the animals using a strategy where the females were introduced into a male cage. Once a female was impregnated, she was returned to her original cage. After two and a half weeks the animals were sacrificed, their uteri were removed, and the number of fetuses was counted.

From Table 2 it can be inferred that lactate dehydrogenase-C produced about 50% infertility in mice, which is consistent with previous fertility studies where purified lactate dehydrogenase-C from testes was used as an injectable antigen. Two of the animals in the plant lactate dehydrogenase-C group were completely sterile. In other two, their fertility was significantly reduced to eight and seven fetuses. The last two animals did not showed any infertility since they had a litter size that resembled the control group. As previous studies indicate, there was not an easily observed correlation between antibody production and infertility. It can be concluded, however, that a plant-produced antigen can serve as an immunocontraceptive agent capable of providing sterility in female mice.

**TABLE 2**

| Fertility of Mice Vaccinated With Plant Lactate Dehydrogenase-C | | | |
|---|---|---|---|
| Group | Total females tested | Total fetuses | Fetuses/female |
| Plant LDH-C | 6 | 32 | 5.3 |
| Control | 3 | 31 | 10.3 |

### EXAMPLE 14

### Plant Vaccine As Immunocontraceptive

Data presented above indicate that lactate dehydrogenase-C gene can be expressed at acceptable levels in both *Arabidopsis* and tobacco. But higher expression levels might be required in the future for dose optimization and lowering the production costs. There are many strategies that are being developed by several research groups to increase the expression of heterologous antigens in transgenic plants. One such strategy is the development of mutant forms of commonly used promoters such as the Cauliflower Mosaic Virus promoter to make "superpromoters" (Tuboly et al., 2000). Other plausible approaches to increase the production of heterologous proteins include fusing the antigens to proteins that are commonly expressed in large amounts in plants, such as the alfalfa beta amylase gene (Tuboly et al., 2000) and changing the nucleotide sequence of the gene so that it is optimized for plant codon usage. Another alternative approach is to express the antigenic genes via integration into the chloroplast genome. It has been shown that this technique can yield plants capable of expressing the heterologous gene up to 47% of the total soluble protein (Daniell et al., 2001). In conclusion, an ever-increasing number of techniques are being developed to increase production of heterologous genes in plants.

It is important to note that purifying lactate dehydrogenase-C to homogeneity and injecting it emulsified in an adjuvant showed that plant-produced lactate dehydrogenase-C is capable of inducing immunity and sterility. A more desirable approach would be to engineer edible plants such as potato, corn, or banana to produce mouse lactate dehydrogenase-C. Potato is a particular good candidate since it can be transfected relatively easily, the tuber can be eaten raw by animals, and present low potential for outcrossing since it propagates clonally (Mason et al., 2002). It has been shown that "doses" of raw potato in the presence of a mucosal adjuvant (cholera toxin) are capable of granting protective immunity to hepatitis antigens.

An issue of concern with plant vaccines and oral vaccines in general is the requirement of strong mucosal adjuvants such as cholera toxin or heat labile enterotoxin from *E. coli.* Such agents are necessary for breaking immune tolerance. The inclusion of such adjuvants might be a difficult and costly endeavor for wildlife contraceptives. However, both the B subunits of cholera toxin and the heat labile enterotoxin have been expressed in several plant systems (review by Mason et al., 2002). Therefore, making a chimeric protein consisting of cholera toxin (or the heat labile enterotoxin) with either the complete lactate dehydrogenase-C sequence or, better yet, a species-specific immunocontraceptive peptide, would be a desirable strategy to pursue. This chimeric protein has the potential of binding to epithelial cells and entering the cells without harming them or producing diarrhea the toxin component lacks the catalytic A-subunit. Overall, the data of immune responses seen in animals injected with purified lactate dehydrogenase-C from transgenic plant can be considered solid and of high quality.

The following references were cited herein:
Babson and Babson, *Clin Chem.* 19:766-9 (1973).
Benfey et al., *EMBO J.* 9:1677-84 (1990).
Daniell et al., *Trends Plant Sci.* 6:219-26 (2001).
Fagard and Vaucheret, *Annu Rev Plant Physiol Plant Mol Biol* 51:167-194 (2000).
Fujihashi et al., *Vaccine.* 20:2431-8 (2002).
Goldberg, *J Biol Chem* 247:2044 (1972).
Goldberg, *Science* 181:458 (1973).
Goldberg et al., *Fertil Steril.* 35:214 (1981).
Haq et al., *Science.* 268:714-6 (1995).
Hogrefe et al., *J Biol Chem.* 262:13155-62 (1987).
Horsch et al., *Science.* 227:1229-31 (1985).
Kirkpatrick et al., *Reprod Fertil*. 9:105-10 (1997).
Koncz and Schell, *Mol Gen Gene.* 204:383-96 (1986).
Lerum and Goldberg, *Biol Reprod.* 11:108 (1974).
Lessard et al., *Metab Eng*. 4:67-79 (2002).
Mason et al., *Proc Natl Acad Sci USA.* 89:11745-9 (1992).
Mason et al.,. *Trends Mol Med* 8:324-9 (2002).
Newell, *Mol Biotechnol.* 16:53-65 (2000).
O'Hern et al., *Biol Reprod.* 52:331-39 (1995).
O'Hern et al., *Vaccine.* 16:1761-6 (1997).
Pizza et al., *Vaccine* 21:2534-41 (2001).
Schagger and von Jagow, *Anal Biochem.* 166:368-79 (1987).
Schardl et al., *Gene* 61:1-11 (1987).
Smith et al., *Reprod Fertl Dev.* 9:85-9 (1997).
Tuboly et al., *Vaccine.* 18:2023-8 (2000).
Van Hareen and Houck, *Plant Mol Bio.* 21:625-640 (1993).
Van Regenmortel et al. *Immunol Lett.* 17:95-107 (1998).
Wheat et al., *Mol Immun.* 22:1195-9 (1985).

Any patents or publications mentioned in this specification are indicative of the levels of those skilled in the art to which the invention pertains. Further, these patents and publications are incorporated by reference herein to the same extent as if each individual publication was specifically and individually indicated to be incorporated by reference.

## Claims

1. A genetically modified plant that expresses an immunocontraceptive comprising an egg-specific polypeptide or sperm-specific polypeptide.

2. The genetically modified plant of claim 1, wherein said sperm-specific polypeptide is lactate dehydrogenase-C or an antigenic fragment thereof.

3. The genetically modified plant of claim 2, wherein said lactate dehydrogenase-C is a rodent lactate dehydrogenase-C.

4. The genetically modified plant of claim 3, wherein said rodent lactate dehydrogenase-C is a rat or mouse lactate dehydrogenase-C.

5. The genetically modified plant of claim 2, wherein said antigenic fragment is selected from the group consisting of amino acids 5-17, 44-58, 61-77, 97-110, 180-210, 211-220, 231-243, 283-306, 307-316, and 101-115 of murine lactate dehydrogenase-C.

6. The genetically modified plant of claim 1, wherein said plant is selected from the group consisting of potato, tobacco, corn, banana, wheat, rice, fruit, vegetable, legume, and grain crop.

7. A method of decreasing the fertility of an animal, comprising the step of:
providing said animal the genetically modified plant of claim 1 thus allowing said animal to ingest the plant.

8. The method of claim 7, wherein said animal is selected from the group consisting of mouse, rat, deer, elephants, water buffalo, feral horses, foxes, urban or wild dogs, urban or wild cats, rabbits, and other potentially overpopulated species causing economic damage to society.

9. A genetically modified plant that expresses a sperm-specific polypeptide lactate dehydrogenase-C or an antigenic fragment thereof.

10. The genetically modified plant of claim 9, wherein said lactate dehydrogenase-C is a rodent lactate dehydrogenase-C.

11. The genetically modified plant of claim 10, wherein said rodent lactate dehydrogenase-C is a rat or mouse lactate dehydrogenase-C.

12. The genetically modified plant of claim 9, wherein said antigenic fragment is selected from the group consisting of amino acids 5-17, 44-58, 61-77, 97-110, 180-210, 211-220, 231-243, 283-306, 307-316, and 101-115 of murine lactate dehydrogenase-C.

13. The genetically modified plant of claim 9, wherein said plant is selected from the group consisting of potato, tobacco, corn, banana, wheat, rice, fruit, vegetable, legume, and grain crop.

14. A method of decreasing the fertility of an animal, comprising the step of:
allowing said animal to ingest the genetically modified plant of claim 9.

15. The method of claim 14, wherein said animal is selected from the group consisting of mouse, rat, deer, elephants, water buffalo, feral horses, foxes, urban or wild dogs, urban or wild cats, rabbits, and other potentially overpopulated species causing economic damage to society.


### Annex to the application documents - subsequently filed sequences listing

SEQUENCE LISTING

<110> RESEARCH DEVELOPMENT FOUNDATION
      KITTO, G. BARRIE
      HIRSCHHORN, DANIEL C.

<120> ANIMAL IMMUNOCONTRACEPTIVES EXPRESSED IN PLANTS
      AND USES THEREOF

<130> F63408EP

<140> 05004437.9
<141> 2004-03-01

<150> 10/664,118
<151> 2003-09-17

<150> 60/412,043
<151> 2002-09-19

<160> 11

<170> PatentIn Ver. 2.1

<210> 1
<211> 19
<212> DNA
<213> Artificial Sequence

<220>
<223> Description of Artificial Sequence:  Synthetic
      Primer

<400> 1
aggacacgtg aaatcacca                                              19


<210> 2
<211> 34
<212> DNA
<213> Artificial Sequence

<220>
<223> Description of Artificial Sequence:  Synthetic
      Primer

<220>
<221> modified_base
<222> (1)..(5)
<223> n = a, c, g and/or t/u

<400> 2
nnnnnggatc ctactataac tgcacatcct tctg                             34


<210> 3
<211> 13

<212> PRT
<213> Mus musculus

<400> 3
Gln Leu Ile Asn Leu Val Pro Glu Asp Lys Leu Ser Arg
1               5                   10

<210> 4
<211> 13
<212> PRT
<213> Rattus norvegicus

<400> 4
Gln Leu Ile Asn Leu Ala Pro Asp Glu Lys Gln Ser Arg
1               5                   10

<210> 5
<211> 13
<212> PRT
<213> Homo sapiens

<400> 5
Gln Leu Ile Lys Leu Ile Glu Asp Asp Glu Asn Ser Gln
1               5                   10

<210> 6
<211> 13
<212> PRT
<213> Hamster sp.

<400> 6
Gln Leu Ile Asn Leu Thr Gln Glu Asp Lys Thr Ser Arg
1               5                   10

<210> 7
<211> 13
<212> PRT
<213> Sus scrofa

<400> 7
Gln Leu Ile Asn Leu Ile Glu Asp Glu Val Ser Gln Arg
1               5                   10

<210> 8
<211> 13
<212> PRT
<213> Fox

<400> 8
Gln Leu Glu Asn Leu Ile Glu Glu Asp Lys Ile Ser Gln
1               5                   10

<210> 9
<211> 13
<212> PRT
<213> Pigeon

<400> 9
Gln Leu Ile Met Thr Pro Ile Thr Ala Gln Ile Lys Val
1               5                   10

<210> 10
<211> 13
<212> PRT
<213> Fundulus heteroclitus

<400> 10
His Lys Leu Ile Thr Pro Leu Ala Cys Ser Ser Pro Glu
1               5                   10

<210> 11
<211> 13
<212> PRT
<213> Frog

<400> 11
Asn Leu Ile Thr Asn Val Cys Gln Asp Lys Ala Ala Ala
1               5                   10